(19) Europäisches Patentamt
European Patent Office
Office européen des brevets

(11) Publication number: **0 105 763**
**B1**

(12) **EUROPEAN PATENT SPECIFICATION**

(45) Date of publication of patent specification: **20.01.88**

(21) Application number: **83306030.4**

(22) Date of filing: **05.10.83**

(51) Int. Cl.$^4$: **C 07 D 209/12,** C 07 D 209/42, A 61 K 31/40

(54) **Indole compounds, their preparation and pharmaceutical compositions comprising them.**

(30) Priority: **05.10.82 JP 173949/82**

(43) Date of publication of application: **18.04.84 Bulletin 84/16**

(45) Publication of the grant of the patent: **20.01.88 Bulletin 88/03**

(84) Designated Contracting States: **DE FR GB IT SE**

(56) References cited:
**US-A-4 152 446**

**VERLAG CHEMIE, Weinheim/Bergstr., vol.2, 2nd edition, 1972, G. EHRHART et al.: "Arzneimittel. Therapeutica mit Wirkung auf das periphere Nervensystem, pp. 195-198, 207.**

(73) Proprietor: **KOWA COMPANY, LTD.**
**6-29, 3-chome Nishiki**
**Naka-ku Nagoya-shi Aichi-ken (JP)**

(72) Inventor: **Shiratsuchi, Masami**
**4-43-2, Zanbori**
**Musashimurayama-shi Tokyo (JP)**
Inventor: **Onogi, Kazuhiro**
**457-19, Ohgimachiya**
**Iruma-shi Saitama-ken (JP)**
Inventor: **Ishimaha, Hiroshi**
**2-8-8, Suwa-cho**
**Higashimurayama-shi Tokyo (JP)**

(74) Representative: **Myerscough, Philip Boyd et al**
**J.A.Kemp & Co. 14, South Square Gray's Inn**
**London, WC1R 5EU (GB)**

**EP 0 105 763 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel indole compounds having various pharmacological activities such as blood pressure lowering activity, vasodilating activity, adrenergic beta-blocking activity and blood flow increasing activity. The invention also relates to processes for the production of the aforesaid compounds.

US—A—4152446 discloses aminopropanol compounds of the formula

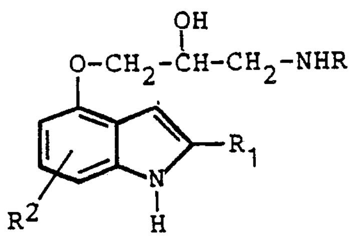

where R is alkyl, cycloalkyl or alkylthioalkyl, $R_2$ is hydrogen, alkyl, hydroxyalkyl, alkoxyalkyl or pivaloylalkyl and among eight possibilities for $R_1$ is included $—CONR_3R_4$ where $R_3$ and $R_4$ are hydrogen or lower alkyl. The compounds are said to be effective in the treatment of cardiac and circulatory diseases. Among the many compounds mentioned are two in which R is alkylthioalkyl and $R_1$ is $CONR_3R_4$, with $R_3$ and $R_4$ both being either hydrogen (Example 8) or methyl (Example 9). No test data are given for these two compounds.

Arzneimittel, 2nd Edition, Vol. 2 (1972), 195—198 and 207 discloses the use of various nitrites and nitrates, including isosorbide dinitrates, as coronary vasodilators. Moderne Arzneimittel, 5 Auflage 1980, page 855 suggests the use of a mixture of isosorbide dinitrate with 1-(indol-4-yloxy)-3-isopropylaminopropan-2-ol (a beta-blocker) in the treatment of cardiac disease.

The compounds of this invention have not been described in the literature and are novel. They can be represented by the following formula (I).

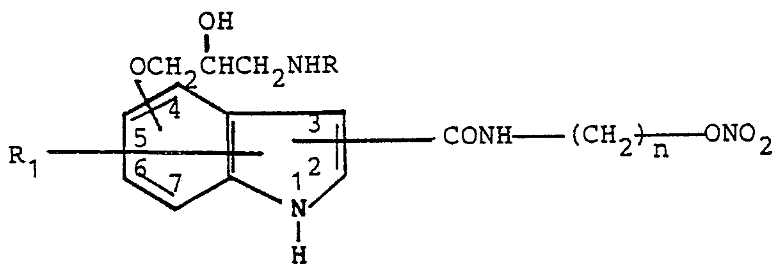
..... (I)

wherein R represents a linear or branched $C_2—C_5$ alkyl group which may be substituted by a phenyl or phenoxy group unsubstituted or substituted by a $C_1—C_3$ alkoxy group, $R_1$ represents a hydrogen atom, a $C_1—C_3$ alkyl group, or a nitro group, and n is an integer of 1 to 5.

In formula (I), example of the $C_2—C_5$ alkyl group are ethyl, n- or iso-propyl, and n-, iso-, sec- or tert-butyl. The alkyl group may be substituted by a phenyl or phenoxy group unsubstituted or substituted by a $C_1—C_3$ alkoxy group, preferably a methoxy group. $R_1$ in formula (I) may be bonded not only to the 4-, 5-, 6- or 7-position of the indole ring, but also to the 2- or 3-position. The symbol *n* preferably represents an integer of 2 or 3.

Specific examples of the compound of formula (I) are:

4- or 5-[(2-hydroxy-3-n- or iso-propylamino)propyloxy]-N-(2- or 3-nitroxyethyl or -nitroxypropyl)indole-2-carboxamide,

4- or 5-[(2-hydroxy-3-n-, iso-, sec- or tert-butylamino)propyloxy]-N-(2- or 3-nitroxyethyl or -nitroxypropyl)indole-2-carboxamide,

4- or 5-[(2-hydroxy-3-beta-phenethylamino)propyloxy]-N-(2- or 3-nitroxyethyl or -nitroxypropyl)indole-2-carboxamide,

4- or 5-{[(2-hydroxy-3-(beta-methoxyphenoxy)ethylamino]propyloxy}-N-[2- or 3-nitroxyethyl or -nitroxypropyl)indole-2-carboxamide,

4- or 5-[(2-hydroxy-3-amylamino)propyloxy]-N-(2- or 3-nitroxyethyl or -nitroxypropyl)indole-2-carboxamide, and

4- or 5-[(2-hydroxy-3-n- or iso-propylamino)propyloxy]-7-nitro-N-(2- or 3-nitroxyethyl or -nitroxypropyl)indole-2-carboxamide.

The compounds of formula (I) can be produced, for example, by the following process (a), (b), (c) or (d).

Process (a)

A compound of the following formula (II)

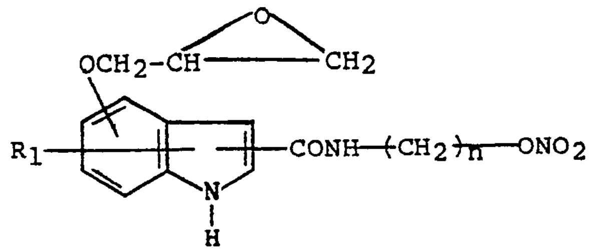 (II)

wherein $R_1$ and n are as defined hereinabove, is reacted with an amine compound of the following formula (III)

$$H_2N—R \qquad (III)$$

wherein R is as defined above.

Process (b)

A compound of the following formula (IV)

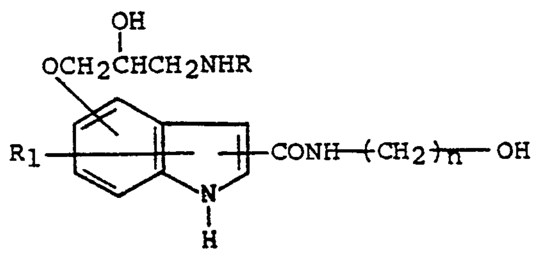 (IV)

wherein R, $R_1$ and n are as defined hereinabove, is subjected to a nitrate ester-forming reaction.

Process (c)

A compound of the following formula (V)

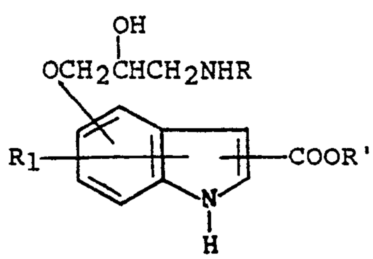 (V)

wherein R and $R_1$ are as defined above, and R′ represents a hydrogen atom or a $C_1$—$C_3$ alkyl group, is reacted with a compound of the following formula (VI)

$$H_2N{-}(CH_2)_n{-}ONO_2 \qquad (VI)$$

wherein n is as defined above.

Process (d)

A compound of the following formula (VIII)

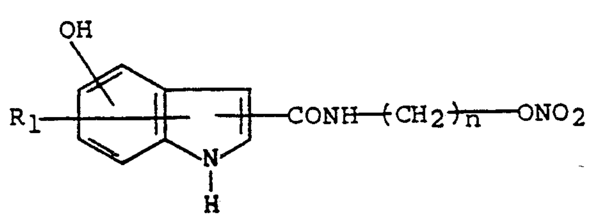 (VIII)

wherein $R_1$ and n are as defined hereinabove,
is subjected to an aminoalkanol-forming reaction.

The compound of formula (II) used in process (a) can be produced by subjecting to a nitrate ester-forming reaction a compound of the following formula (X)

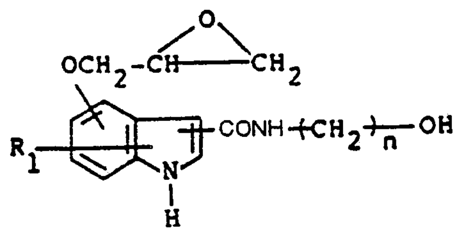

..... (X)

wherein $R_1$ and n are as defined above,
which can be obtained by subjecting a compound of the following formula (IX)

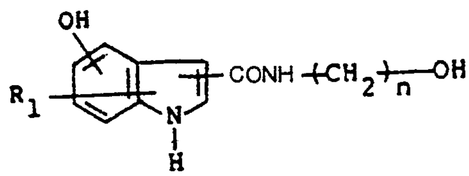

.... (IX)

wherein $R_1$ and n are as defined above,
to a glycidyl-forming reaction. The compound of formula (II) can also be produced by subjecting to a glycidyl-forming reaction the compound of formula (VIII) which can be obtained by subjecting the compound of formula (IX) to a nitrate ester-forming reaction.

A compound of formula (VIII) can be produced by reacting a hydroxyindole carboxylic acid derivative of the following formula (XI)

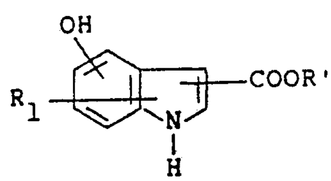

.... (XI)

wherein $R_1$ and R′ are as defined above,
with the compound of formula (VI). The compound of formula (VIII) which can be so produced may also be utilized as a starting compound in process (d).

The compound of formula (IX) can be obtained by protecting the OH group of the compound of formula (XI) and then reacting the protected compound of formula (XI) with a compound of the following formula (VII)

$$H_2N-(CH_2)_n-OH \qquad (VII)$$

wherein n is as defined above.

The compound of formula (IV) used in process (b) can be produced by reacting the compound of formula (X) with the amine compound of formula (III). A compound of formula (IV) can be produced also by reacting the compound of formula (V) with the compound of formula (VII).

The compound of formula (V) which can be used in the production of the compound of formula (IV) and also as a starting material in process (c) can be produced by reacting a compound of the following formula (XII)

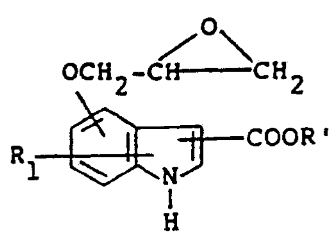

..... (XII)

wherein R and R′ are as defined above,

which can be obtained by subjecting the compound of formula (XI) to a glycidyl-forming reaction, with the amine compound of formula (III).

The processes (a) to (d) are schematically shown below together with examples of producing the starting compounds used therefor.

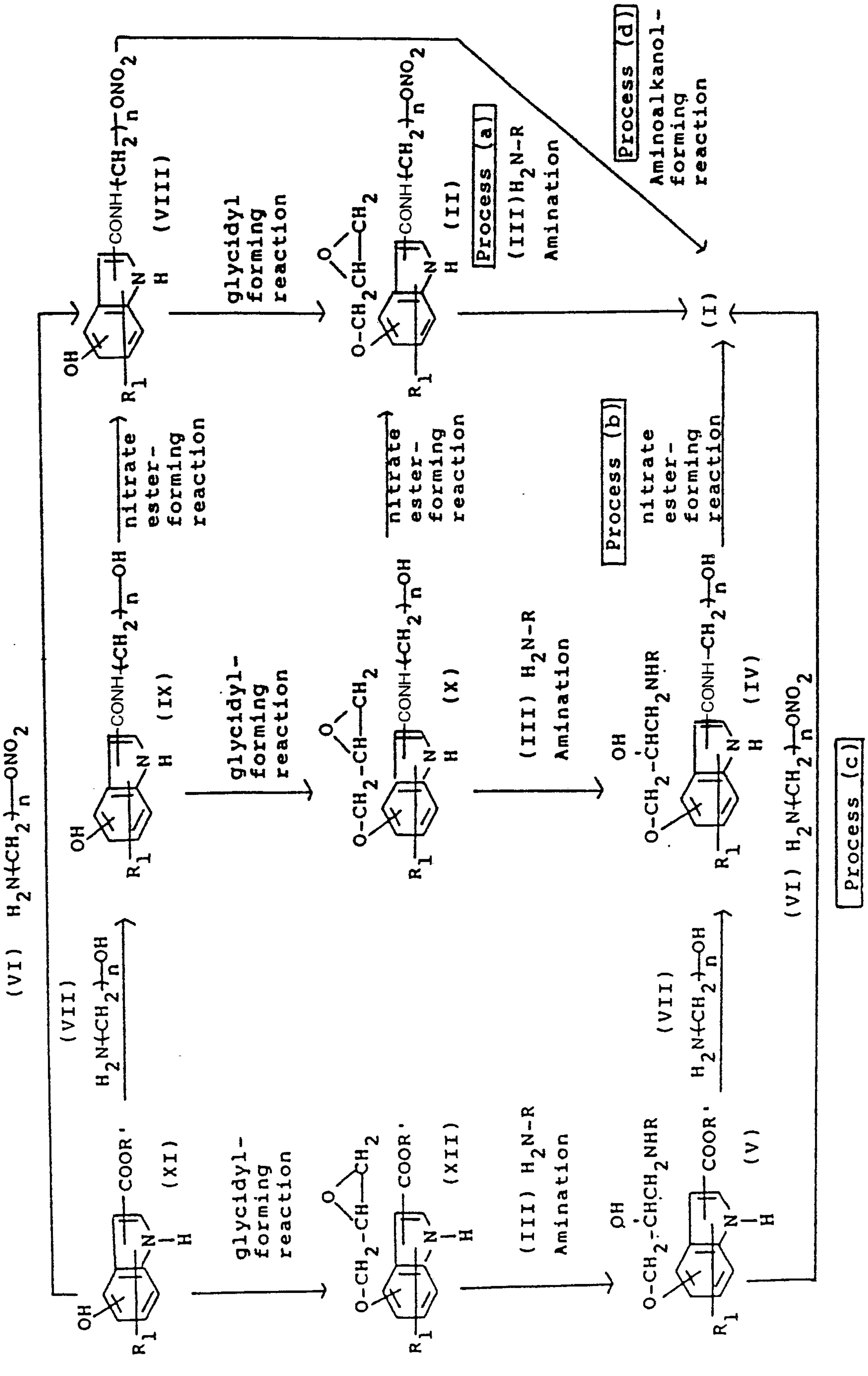

(XI)
(XII)
(V)
(IX)
(X)
(IV)
(VIII)
(II)
(I)
glycidyl-forming reaction
glycidyl forming reaction
(III) $H_2N$-R Amination
(VII) $H_2N\!+\!CH_2\!\!+_n\!OH$
(VI) $H_2N\!+\!CH_2\!\!+_n\!ONO_2$
nitrate ester-forming reaction
Process (a)
(III) $H_2N$-R Amination
Process (b)
Process (c)
Process (d)
Aminoalkanol-forming reaction

In processes (a) to (d) and the production processes for starting compounds in these processes, the amination with the amine compound of formula (III), the amidation with the compound of formula (VI), the amidation with the compound of formula (VII), the nitrate ester-forming reaction, the amino-alkanol-forming reaction, and the glycidyl-forming reaction can be performed as shown below.

1. Amination

The amination of the epoxy group-containing compound of formula (II), (X) or (XII) with the compound of formula (III) can be carried out by contacting the compound of formula (II), (X) or (XII) with the compound of formula (III) in a suitable solvent. The solvent may be an inert organic solvent. Specific examples include methanol, ethanol and isopropanol. The reaction is carried out preferably under heating, for example under refluxing temperature conditions, for example at a reaction temperature ranging from room temperature to about 150°C. The reaction time can be selected properly, and is, for example, about 1 to about 5 hours.

2. Nitrate ester-forming reaction

The nitrate ester-forming reaction of the hydroxyalkylamide derivative of formula (IV), (IX) or (X) can be carried out by contacting the compound of formula (IV), (IX) or (X) with a nitrating reagent such as fuming nitric acid or a mixture of fuming nitric acid and acetic anhydride in the presence or absence of a solvent. Examples of the solvent that may be used include acetonitrile, dioxane and tetrahydrofuran. Preferably, the reaction is carried out at relatively low temperatures, for example at about −40°C to room temperature. The reaction time can be properly selected, and is, for example, several minutes to about 1 hour. When the compound of formula (VIII) is to be produced from the compound of formula (IX), the nitrate ester-forming reaction is carried out after the hydroxyl group excepting $-CONH-(CH_2)_n-OH$ is protected. Specific protective groups used for the protecting reaction include a trichloroacetyl group, a dichloroacetyl group and a trichloroacetyl group.

3. Amidation

When the compound of formula (XI) or (V) is an indole carboxylic acid ester in which R′ in the group —COOR′ is a $C_1$—$C_3$ alkyl group, the amidation of the compound of formula (XI) or (V) with the nitroxyalkylamine of formula (VI) or the hydroxyalkylamine of formula (VII) can be carried out by contacting this ester with the compond of formula (VI) or (VII). The reaction temperature can be properly chosen and is, for example, room temperature to about 130°C. The reaction time can also be properly chosen, and is, for example, about 1 to about 6 hours. When R′ in the group —COOR′ in the compound of formula (XI) or (V) is a hydrogen atom (namely, when the group —COOR′ is a free carboxyl group), the amidation reaction can be carried out by contacting the compound of formula (XI) or (V) with the compound of formula (VI) or (VII) in the presence of a suitable coupling reagent such as dicyclohexyl carbodiimide or carbonyl diimidazole at a relatively low temperature of, for example, about 0 to about 70°C.

In the reaction of the compound of formula (XI) with the compound of formula (VI) or (VII), the reaction is carried out preferably after the OH group other than —COOR′ (R′=H) in formula (XI) is protected. The protecting reaction can be carried out in the same way as described hereinabove with regard to the nitrate ester-forming reaction in section 2 above.

4. Aminoalkanol-forming reaction

The aminoalkanol-forming reaction of the compound of the formula (VIII) can be carried out by reacting it with an alkylaminoalkanol under ordinary conditions.

5. Glycidyl-forming reaction

The glycidyl-forming reaction of the compound of formula (VIII), (IX) or (XI) can be carried out, for example, by contacting it with epichlorohydrin and piperidine or 1,8-azabicyclo[5.4.0]undec-7-ene (DBU) at a reaction temperature ranging from room temperature to about 100°C for about 1 to about 5 hours.

The novel compounds of formula (I) which can be produced as described above show various pharmacological activities such as blood pressure lowering activity, vasoldilating activity, adrenergic beta-blocking activity and blood flow increasing activity and are useful, for example, as anti-angina pectoris agents, hypotensive agents, cerebral circulation improvers and antiarrhythmic agents.

Accordingly, the present invention can also provide a pharmaceutical composition comprising an amount, effective for the treatment of cardiovascular disease, of a compound of formula (I) and a pharmaceutically acceptable diluent or carrier.

Liquid or solid carriers or diluents may be used in forming the pharmaceutical composition. They may include excipients, binders, lubricants, emulsifiers, etc. known in pharmaceutical production. Examples of these carriers or diluents include starches such as potato starch, wheat starch, corn starch and rice starch; sugars such as lactose, sucrose, glucose, mannitol and sorbitol; celluloses such as crystalline cellulose, calcium carboxymethyl cellulose and hydroxypropyl cellulose of a low degree of substitution; inorganic substances such as potassium phosphate, calcium sulfate, calcium carbonate and talc; binder compounds such as gelatin, gum arabic, methyl cellulose, sodium carboxymethyl cellulose, polyvinyl pyrrolidone and hydroxypropyl cellulose; polyhydric alcohol ester-type nonionic surfactants such as fatty acid monoglycerides, sorbitol fatty acid esters, sucrose and plyglycerol fatty acid esters; and polyoxyethylene-

type nonionic surfactants.

The pharmaceutical composition may be in any dosage forms known in the art of formulating pharmaceuticals, such as suppositories, powders, granules, tablets, sublingual tablets, liquid preparations, injectable preparations, and suspension.

The pharmaceutical composition may be administered through any of peroral or parenteral routes, such as intravenous, sublingual or intrarectal administation. For long-term administration, the oral route is preferred.

The dose may be changed as desired. For example, the compound of formula (I) may be administered in a dose of about 1 to about 100 mg/body/day, preferably about 5 to about 50 mg/body/day. The compounds of this invention have low toxicity.

The compounds of formula (I) in accordance with this invention have been found to be active in the following pharmacological activity tests.

Test 1: Blood pressure and heart rate using dogs.

Test 2: β-Blocking action using isolated atrial muscle of a guinea pig.

Test 3: Coronary blood flow using dogs.

Example 1

5-[(2-Hydroxy-3-isopropylamino)propyloxy]-N-(3-nitroxypropyl)indole-2-carboxamide of the formula:

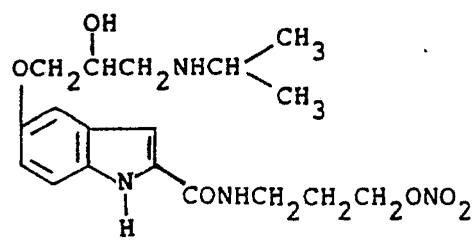

(1) A mixture of 10.57 g of ethyl 5-benzyloxyindole-2-carboxylate and 16.80 g of 3-aminopropanol was stirred at 120°C for 3 hours. After cooling, water was added and the mixture was acidified with concentrated hydrochloric acid and then extracted with ethyl acetate. The extract was washed with water, dried and crystallized to give 8.41 g of colorless prismatic crystals having a melting point of 140 to 145°C.

The crystals were dissolved in 350 ml of ethanol, and 0.5 g of 10% palladium-carbon was added. The mixture was stirred for 5 hours under a hydrogen stream. The catalyst was removed by filtration. The reaction mixture was concentrated, and the resulting yellow oil was crystallized from ethyl acetate to give 7.00 g (yield 99.3%) of 5-hydroxy-N-(3-hydroxypropyl)indole-2-carboxamide as colorless prismatic crystals having a melting point of 186 to 188°C.

MS: m/e 234 [$M^+$]

IR, $\nu_{max}^{KBr}$ $cm^{-1}$: 3250 (NH, OH), 1610 (CONH), 1050 (C=O).

(2) Epichlorohydrin (21.65 g) was added to a solution of 2.74 g of the resulting 5-hydroxy-N-(3-hydroxypropyl)indole-2-carboxamide and 3.56 g of 1,8-diazabicyclo[5.4.0]undec-7-ene (DBU) in 100 ml of methanol, and the mixture was stirred under refluxing for 3 hours. After the reaction, the solvent was evaporated under reduced pressure. An aqueous solution of sodium chloride was added to the resulting oily product, and the mixture was extracted with chloroform. The extract was washed with water and dried and the solvent was evaporated. The residue was crystallized from ethyl acetate to give 1.06 g (yield 31.3%) of 5-(2,3-epoxypropyloxy)-N-(3-hydroxypropyl)indole-2-carboxamide as colorless needlelike crystals having a melting point of 99 to 101°C.

(3) A solution of 0.393 g of the resulting epoxy compound in 20 ml of acetonitrile was cooled to −30°C, and 0.496 g of an equimolar mixture of acetic anhydride and fuming nitric acid was added, and the mixture was stirred for 10 minutes. After the reaction, an aqueous solution of sodium bicarbonate was injected into the reaction mixture, and it was extracted with ethyl acetate. The extract was washed with water, dried, and purified by silica gel column chromatography. Crystallization gave 0.073 g (yield 16.0%) of 5-(2,3-epoxypropyloxy)-N-(3-nitroxypropyl)indole-2-carboxamide as yellow prismatic crystals having a melting point of 148 to 149°C.

MS: m/e 335 [$M^+$] 272 [$M^+$—$HONO_2$]

IR, $\nu_{max}^{KBr}$ $cm^{-1}$: 3360 (CONH), 3260 (NH), 1640 (CONH), 1620, 1280 ($ONO_2$).

(4) Methanol (10 ml) and 0.14 g of isopropylamine were added to 0.076 g of the resulting epoxy-nitroxy compound, and the mixture was heated under reflux for 3 hours with stirring. After the reaction, the solvent was evaporated, and the resulting oily product was separated by alumina thin-layer chromatography (developing solvent: chloroformmethanol=10/1) — to give 0.029 g (yield 32.4%) of 5-[(2-hydroxy-3-isopropylamino)propyloxy]-N-(3-nitroxypropyl)indole-2-carboxamide as a pale yellow powder.

MS: m/e 331 [$M^+$—$HONO_2$]
IR, $\nu_{max}^{KBR}$ $cm^{-1}$: 3240 (NH), 1640 (CONH, $ONO_2$), 1280 ($ONO_2$).
$^1$H—NMR: $\delta CDCl_3$:

- 7.33 (1H, d, J=8Hz, HD)
- 7.07 (1H, d, J=2.0Hz, HB)
- 6.96 (2H, d, d, J=8.8Hz, J=2.2Hz, HC)
- 6.91 (1H, d, J=0.7Hz, HA)
- 4.57 (2H, t, J=6.4Hz, —$C\underline{H_2}ONO_2$)
- 4.17—3.92 (3H, m, —$OC\underline{H_2}C\underline{H}$(OH)—)
- 3.53 (2H, t, J=6.6Hz, —$CONHC\underline{H_2}$—)
- 2.94—2.63 (3H, m, —$C\underline{H_2}NHC\underline{H}$< )
- 2.07 (2H, q, J=6.6Hz, —$CH_2C\underline{H_2}CH_2$—)
- 1.12 (6H, d,d, J=6.5Hz, J=1.5Hz, —CH($C\underline{H_3}$)($C\underline{H_3}$) )

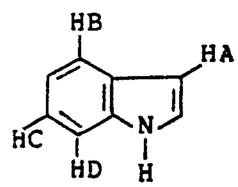

The compounds in the following Examples were synthesized in the same way as in Example 1.

## Example 2

5-[3-t-Butylamino-2-hydroxy)propyloxy]-N-(3-nitroxypropyl)indole-2-carboxamide of the formula:

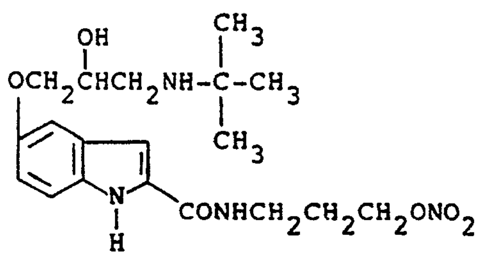

Property: Pale yellow prismatic crystals
Melting point: 129—131°C
MS: m/e 345 [$M^+$&$HONO_2$]
IR, $\nu_{max}^{KBr}$ $cm^{-1}$: 3280 (NH, OH), 1630 (CONH, $ONO_2$), 1280 ($ONO_2$).
$^1$H—NMR: $\delta CDCl_3$:

- 9.11 (1H, br.s, —$CH_2N\underline{H}$—)
- 7.32 (1H, d, J=8.8Hz, HD)
- 7.06 (1H, d, J=2.2Hz, HB)
- 6.99 (1H, d,d, J=8.8Hz, J=2.2Hz, HC)
- 6.75 (1H, s, HA)
- 6.34 (1H, br.s, —$CON\underline{H}$—)
- 4.59 (2H, t, J=6.3Hz, —$C\underline{H_2}ONO_2$)
- 4.08—3.92 (3H, m, —$OC\underline{H_2}\underline{H}$(OH)—)
- 3.61 (2H, q, J=6.4Hz, —$CH_2C\underline{H_2}CH_2O$—)

2.97—2.67 (2H, m, —$C\underline{H_2}N$ )

2.10 (2H, q, J=6.4Hz, —$CONHC\underline{H_2}CH_2$—)
1.14 (9H, s, —$C(CH_3)_3$)

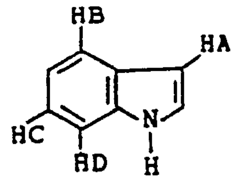

Example 3

5-[(2-Hydroxy-3-beta-phenethylamino)propyloxy]-N-(3-nitroxypropyl)indole-2-carboxamide of the formula:

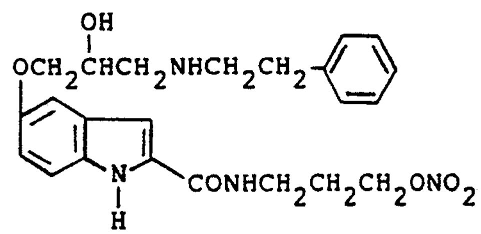

Property: Pale yellow powder
Melting point: 134—136°C
MS: m/e 393 [$M^+$—$HONO_3$] 302 [$M^+$—$HONO_3$, $C_7H_7$]
IR, $\nu_{max}^{KBR}$ $cm^{-1}$: 3300 (OH), 3240 (NH), 1640 (CONH), 1620, 1280 ($ONO_2$).
$^1H$—NMR: $\delta CDCl_3$—$CD_3OD$
7.40—6.87 (9H, m, aromatic ring H)
4.57 (2H, t, J=6.3Hz, —$\underline{CH_2}ONO2$)

OH
4.25—3.94 (5H, m, —$O\underline{CH_2}\underline{CH}$—$CH_2NHCH_2\underline{CH_2}$—)
3.52 (2H, t, J=6.8Hz, —$N\underline{H}\underline{CH_2}$—)
3.05—2.73 (4H, m, —$\underline{CH_2}NH\underline{CH_2}$—)
2.07 (2H, q, J=6.4Hz, —$CH_2\underline{CH_2}CH_2$—)

Example 4

5-[{[2-Hydroxy-3-(beta-methoxyphenoxy)ethylamino]propyloxy}-N-(3-nitroxypropyl)indole-2-carboxamide of the formula:

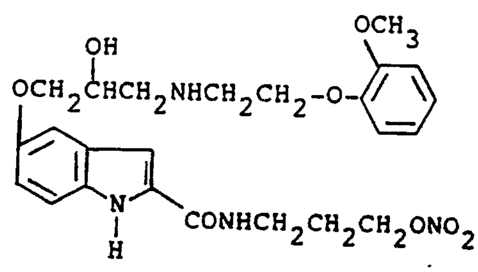

Property: Pale yellow powder
Melting point: 128—131°C
MS: m/e 439 [$M^+$—$HONO_3$]
IR, $\nu_{max}^{KBR}$ $cm^{-1}$: 1620 (CONH, $ONO_2$), 1280 ($ONO_2$).
$^1H$—NMR: $\delta CDCl_3$—$CD_3OD$
7.33 (1H, d, J=8.8Hz, HD)
7.08 (1H, d, J=2.2Hz, HB

7.00—6.77 (6H, m, HA, HB, 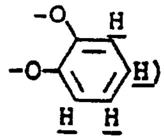

4.57 (2H, t, J=6.6Hz, —$CH_2ONO_2$)

OH
|
4.28—3.64 (5H, m, —$OCH_2CHCH_2NHCH_2CH_2$—)
3.85 (3H, s, —$OCH_3$)
3.53 (2H, t, J=6.6Hz, —$CONHCH_2$—)
3.23—2.89 (4H, m, —$CH_2NHCH_2$—)
2.07 (2H, q, J=6.9Hz, —$CH_2CH_2CH_2$—)

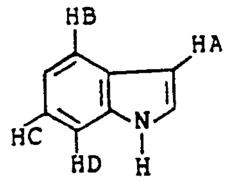

Example 5

4-[(2-Hydroxy-3-isopropylamino)propyloxy-7-nitro-N-(3-nitroxypropyl)indole-2-carboxamide of the formula:

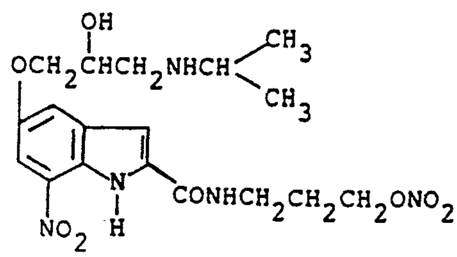

Property: Orange powder
Melting point: 158—160°C
MS: m/e 376 [$M^+$—$HONO_3$]
IR, $\nu_{max}^{KBr}$ $cm^{-1}$: 1630 (CONH), 1620 ($ONO_2$), 1560 ($NO_2$), 1280 ($ONO_2$)
$^1H$—NMR: $\delta CDCl_3$
8.15 (1H, d, J=9.3Hz, HC)
7.39 (1H, s, HA)
6.67 (1H, d, J=9.0Hz, HB)
4.64 (2H, t, J=6.4Hz, —$CH_2ONO_2$)

OH
|
3.95—3.78 (1H, m, —$OCH_2C3H$—$CH_2$—)
3.70—3.45 (7H, m, —$CONHCH_2$—,

OH
|
—$OCH_2CHCH_2NHCH$ ⟨ )

2.10 (2H, q, J=6.4Hz, —$CH_2CH_2CH_2$—)

$CH_3$
/
1.38—1.35 (6H, d,d, J=6.6Hz, —CH )
\
$CH_3$

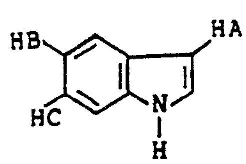

**Claims**

1. A substituted indole compound of formula (I)

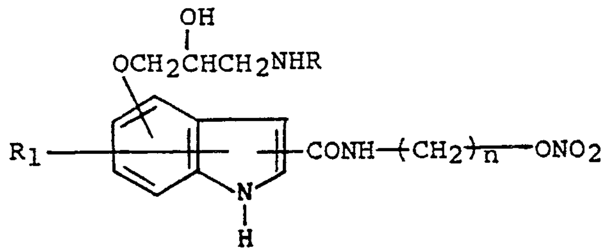

(I)

wherein R represents a linear or branched $C_2$—$C_5$ alkyl group which may be substituted by a phenyl or phenoxy group unsubstituted or substituted by a $C_1$—$C_3$ alkoxy group, $R_1$ represents a hydrogen atom, a $C_1$—$C_3$ alkyl group or a nitro group and n is an integer of 1 to 5.

2. A compound according to claim 1 which is 5-[2-hydroxy-3-isopropylamino)propyloxy]-N-(3-nitroxypropyl)indole-2-carboxamide.

3. A compound according to claim 1 which is 5-[(2-hydroxy-3-tert butylamino)propyloxy]-N-(3-nitroxypropyl)-indole-2-carboxamide.

4. A compound according to claim 1 which is 5-[(2-hydroxy-3-beta-phenethylamino)propyloxy]-N-(3-nitroxypropyl)indole-2-carboxamide.

5. A compound according to claim 1 which is 5-{[2-hydroxy-3-(beta-methoxyphenoxy)ethylamino]-propyloxy}-N-(3-nitroxypropyl)indole-2-carboxamide.

6. A compound according to claim 1 which is 4-[(2-hydroxy-3-isopropylamino)propyloxy]-7-nitro-N-(3-nitroxypropyl)indole-2-carboxamide.

7. A process for the production of a substituted indole compound as claimed in claim 1 which comprises

(a) reacting a compound of formula (II)

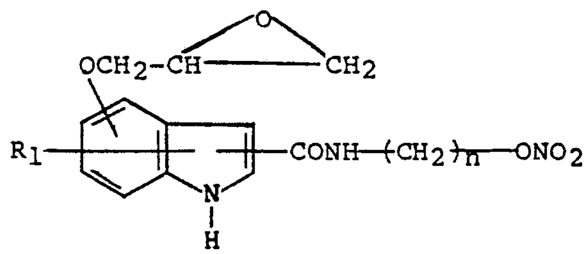

(II)

wherein $R_1$ and n are as defined in claim 1, with an amine compound of formula (III)

$$H_2N—R \qquad (III)$$

wherein R is as defined in claim 1; or

(b) subjecting a compound of formula (IV)

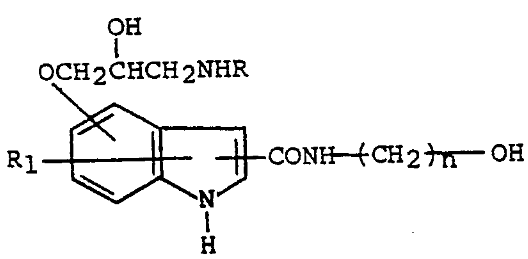

(IV)

wherein R, $R_1$ and n are as defined in claim 1, to a nitrate ester-forming reaction; or

(c) reacting a compound of formula (V)

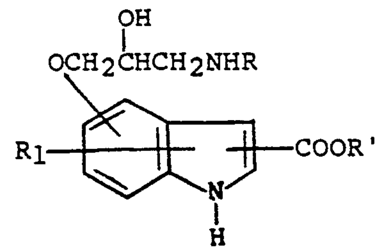

(V)

wherein R and $R_1$ are as defined in claim 1, and R′ represents a hydrogen atom or a $C_1$—$C_3$ alkyl group, with a compound of formula (VI)

$$H_2N-(CH_2)_n-ONO_2 \qquad (VI)$$

wherein n is as defined in claim 1; or

(d) subjecting a compound of formula (VIII)

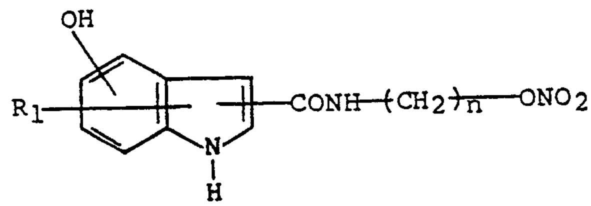

(VIII)

wherein $R_1$ and n are as defined in claim 1, to an aminoalkanol-forming reaction.

8. A pharmaceutical composition comprising, as active ingredient, a substituted indole compound as claimed in any one of claims 1 to 6 in association with a pharmaceutically acceptable diluent or carrier.

**Patentansprüche**

1. Substituierte Indolverbindung der Formel (I)

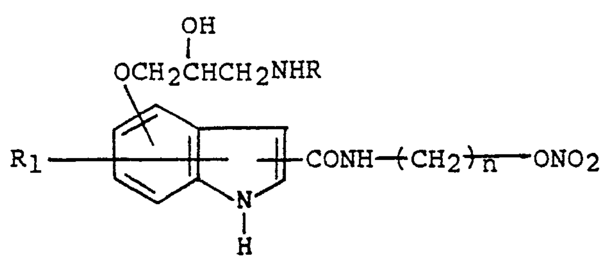

(I)

in der R eine lineare oder verzweigte $C_2$—$C_5$ Alkylgruppe ist, die substituiert sein kann durch eine unsubstituierte oder mit einer $C_1$—$C_3$ Alkoxygruppe substituierten Phenyl- oder Phenoxygruppe, $R_1$ ein Wasserstoffatom, eine $C_1$—$C_3$ Alkylgruppe oder eine Nitrogruppe bedeutet und n eine ganze Zahl von 1 bis 5 ist.

2. Eine Verbindung nach Anspruch 1, die 5-[(2-Hydroxy-3-isopropylamino)propyloxy]-N-(3-nitroxypropyl)indol-2-carboxamid ist.

3. Eine Verbindung nach Anspruch 1, die 5-[(2-Hydroxy-3-tert. butylamino)propyloxy]-N-(3-nitroxypropyl)indol-2-carboxamid ist.

4. Eine Verbindung nach Anspruch 1, die 5-[(2-Hydroxy-3-β-phenethylamino)propyloxy]-N-(3-nitroxypropyl)indol-2-carboxamid ist.

5. Eine Verbindung nach Anspruch 1, die 5-{[2-Hydroxy-3-[β-methoxyphenoxy)ethylamino]-propyloxy}-N-(3-nitroxypropyl)indol-2-carboxamid ist.

6. Eine Verbindung nach Anspruch 1, die 4- 2-Hydroxy-3-isopropylamino)propyloxy-7-nitro-N-(3-nitroxypropyl)indol-2-carboxamid ist.

7. Verfahren zur Herstellung einer substituierten Indolverbindung gemäß Anspruch 1, dadurch gekennzeichnet, daß man

(a) eine Verbindung der Formel (II)

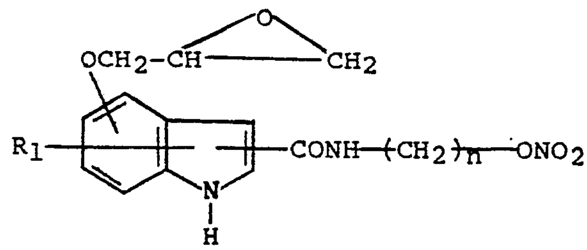 (II)

in der $R_1$ und n wie im Anspruch 1 definiert sind, mit einer Aminverbindung der Formel (III)

$$H_2N—R \qquad \text{(III)}$$

in der R wie im Anspruch 1 definiert ist, umsetzt; oder

(b) eine Verbindung der Formel (IV)

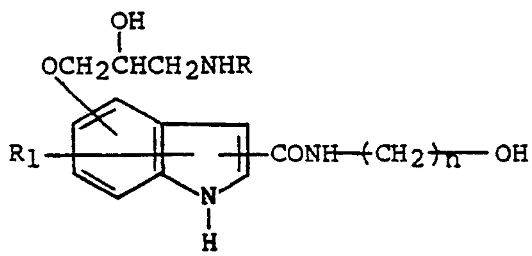 (IV)

in der R, $R_1$ und n wie im Anspruch 1 definiert sind, einer Nitratester-Bildungsreaktion unterwirft; oder

(c) eine Verbindung der Formel (V)

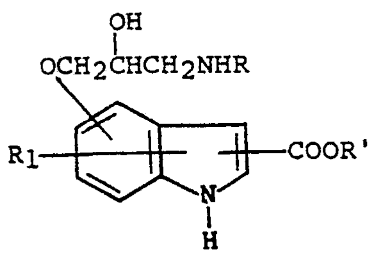 (V)

in der R und $R_1$ wie im Anspruch 1 definiert sind und R' ein Wasserstoffatom oder eine $C_1$—$C_3$ Alkylgruppe bedeutet, mit einer Verbindung der Formel (VI)

$$H_2N-(CH_2)_n-ONO_2 \qquad \text{(VI)}$$

in der n wie im Anspruch 1 definiert ist, umsetzt; oder

(d) eine Verbindung der Formel (VIII)

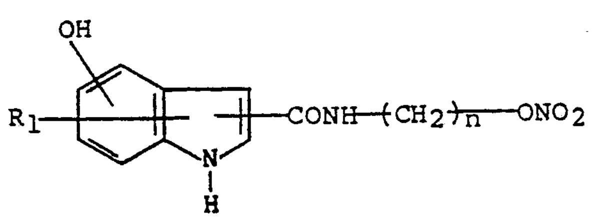 (VIII)

in der $R_1$ und n wie im Anspruch 1 definiert sind, einer Aminoalkanol-Bildungsreaktion unterwirft.

8. Pharmazeutisches Mittel, umfassend als Wirkstoff eine substituierte Indolverbindung nach einem der Ansprüche 1 bis 6 in Kombination mit einem pharmazeutisch annehmbaren Verdünnungsmittel oder Träger.

**Revendications**

1. Composé indole substitué de formule (I):

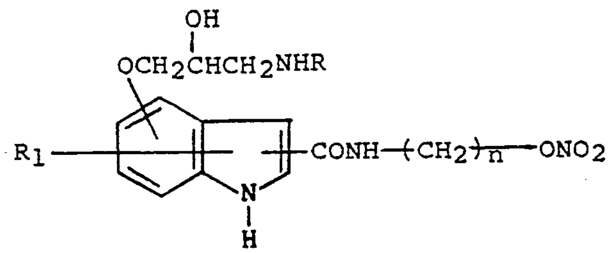

(I)

dans laquelle R représente un groupement alkyle, linéaire ou ramifié en $C_2$—$C_5$ qui peut être substitué par un groupement phényle ou phénoxy non substitué ou substitué par un groupement alcoxy en $C_1$—$C_3$, $R_1$ représente un atome d'hydrogène, un groupement alkyle en $C_1$—$C_3$ ou un groupement nitro et n est un nombre entier compris entre 1 et 5.

2. Composé selon la revendication 1 qui est le 5-[2-hydroxy-3-isopropylamino)propyloxy]-N-(3-nitroxy-propyl)indole-2-carboxamide.

3. Composé selon la revendication 1 qui est le 5-[(2-hydroxy-3-tertiobutylamino)propyloxy]-N-(3-nitroxypropyl)indole-2-carboxamide.

4. Composé selon la revendication 1 qui est le 5-[(2-hydroxy-3-β-phénéthylamino)propyloxy]-N-(3-nitroxypropyl)-indole-2-carboxamide.

5. Composé selon la revendication 1 qui est le 5-([2-hydroxy-3-(β-méthoxyphénoxy)éthylamino]-propyloxy)-N-(3-nitroxypropyl)indole-2-carboxamide.

6. Composé selon la revendication 1 qui est le 4-[(2-hydroxy-3-isopropylamino)propyloxy]-7-nitro-N-(3-nitroxypropyl)indole-2-carboxamide.

7. Procédé de préparation du composé indole substitué selon la revendication 1, qui consiste:

(a) à faire réagir un composé de formule (II):

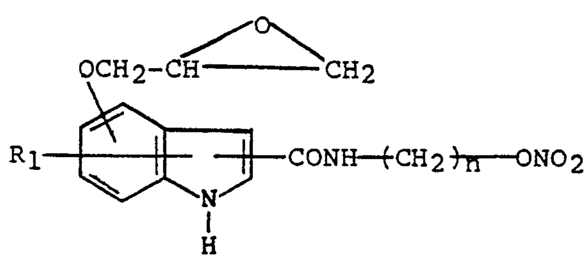

(II)

dans laquelle $R_1$ et n sont tels que définis dans la revendication 1, avec un composé amine de formule (III):

$$H_2N—R \qquad (III)$$

dans laquelle R est tel que défini dans la revendication 1; ou

(b) à soumettre un composé de formule (IV):

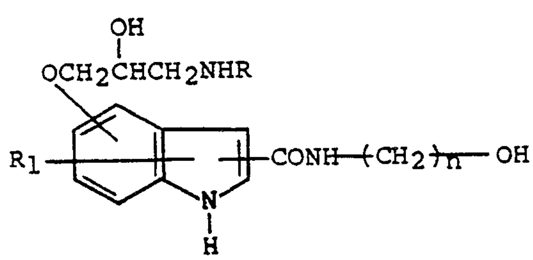

(IV)

dans laquelle R, $R_1$ et n sont tels que définis dans la revendication 1, à une réaction de formation de l'ester nitrate; ou

(c) à faire réagir un composé de formule (V):

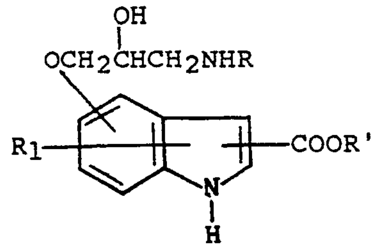

(V)

dans laquelle R et $R_1$ sont tels que définis dans la revendication 1 et R' représente un atome d'hydrogène ou un groupement alkyle en $C_1$—$C_3$ avec un composé de formule (VI):

$$H_2N\text{—}(CH_2)_n\text{—}ONO_2 \qquad (VI)$$

dans laquelle n est tel que défini dans la revendication 1; ou

(d) à soumettre un composé de formule (VIII):

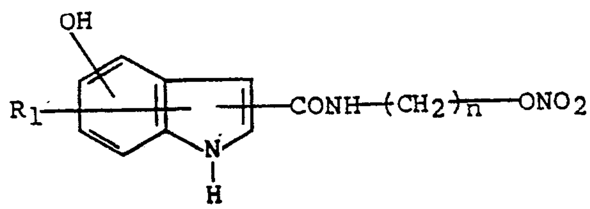

(VIII)

dans laquelle $R_1$ et N sont tels que définis dans la revendication 1, à une réaction de formation d'un amino-alcanol.

8. Composition pharmaceutique comprenant comme principe actif un composé indole substitué tel que revendiqué dans l'une quelconque des revendications 1 à 6, en association avec un support ou diluant pharmacuetiquement acceptable.